(19) Europäisches Patentamt
European Patent Office
Office européen des brevets

(11) Veröffentlichungsnummer: **0 236 973**
**B1**

# (12) EUROPÄISCHE PATENTSCHRIFT

(45) Veröffentlichungstag der Patentschrift: **14.06.89**

(51) Int. Cl.⁴: **C07D 311/58,** C07D 405/06, C07D 417/06, B41M 5/00

(21) Anmeldenummer: **87103198.5**

(22) Anmeldetag: **07.03.87**

## (54) Neue Benzypyrane und deren Verwendung in Aufzeichnungssystemen.

(30) Priorität: **12.03.86 DE 3608215**

(43) Veröffentlichungstag der Anmeldung: **16.09.87 Patentblatt 87/38**

(45) Bekanntmachung des Hinweises auf die Patenterteilung: **14.06.89 Patentblatt 89/24**

(84) Benannte Vertragsstaaten: **CH DE FR GB IT LI**

(56) Entgegenhaltungen:
**FR-A-1 540 458**
**FR-A-2 156 909**

(73) Patentinhaber: **BASF Aktiengesellschaft, Carl-Bosch-Strasse 38, D-6700 Ludwigshafen(DE)**

(72) Erfinder: **Guentner, Andreas, Dr., Carl-Bosch-Ring 20, D-6710 Frankenthal(DE)**
Erfinder: **Mayer, Udo, Dr., Max-Slevogt-Strasse 27, D-6710 Frankenthal(DE)**
Erfinder: **Oberlinner, Andreas, Dr., Bruesseler Ring 53, D-6700 Ludwigshafen(DE)**

EP 0 236 973 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Benzopyrane, die in Ringposition 2 eine Seitenkette aufweisen, ein Verfahren zu ihrer Herstellung sowie ihre Verwendung in druck- oder wärmeempfindlichen Aufzeichnungssystemen.

Es wurden Benzopyrane der allgemeinen Formel I gefunden,

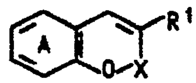

(I)

in der

$R^1$ für gegebenenfalls substituiertes $C_1$-$C_8$-Alkyl, gegebenenfalls substituiertes Phenyl, $C_1$-$C_5$-Alkoxy oder Halogen und

X für die Reste

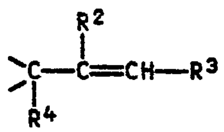

oder

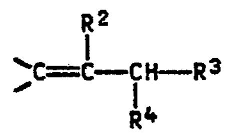

$R^2$ Wasserstoff oder zusammen mit $R^1$ gegebenenfalls durch $C_1$-$C_4$-Alkyl substituiertes $C_2$-$C_3$-Alkylen,

$R^3$ Phenyl, das gegebenenfalls durch $C_1$-$C_4$-Alkyl, das wiederum durch Phenyl substituiert sein kann, Cyclohexyl, Halogen, $C_1$-$C_8$-Alkoxy, $C_1$-$C_8$-Mono- oder Dialkylamino, das jeweils wiederum durch Phenyl oder Chlor substituiert sein kann, Phenylamino, das wiederum durch $C_1$-$C_8$-Alkyl am Phenylring oder am Stickstoffatom substituiert sein kann, Pyrrolidino, Piperidino oder Morpholino substituiert ist, und das gegebenenfalls weiterhin durch $C_1$-$C_5$-Alkyl, $C_1$-$C_5$-Alkoxycarbonyl, $C_1$-$C_5$-Dialkylaminocarbonyl, Pyrrolidinocarbonyl, Piperidinocarbonyl, Morpholinocarbonyl, $C_1$-$C_8$-Alkoxy oder Halogen substituiert ist; gegebenenfalls durch $C_1$-$C_4$-Alkoxy substituiertes Naphthyl; Carbazol-3-yl oder 1,2,3,4,4a,9a-Hexahydrocarbazol-6-yl, das jeweils durch $C_1$-$C_5$-Alkyl oder Benzyl am Stickstoffatom substituiert sein kann, Indol-3-yl, das in Ringposition 1 und/oder 2 durch gegebenenfalls phenylsubstituiertes $C_1$-$C_5$-Alkyl substituiert sein kann; oder Thiazol-5-yl, das in Ringposition 2 durch gegebenenfalls phenylsubstituiertes $C_1$-$C_5$-Mono- oder Dialkylamino, Pyrrolidino, Piperidino oder Morpholino und in Ringposition 4 gegebenenfalls durch $C_1$-$C_5$-Alkyl, Phenyl oder Chlor substituiert ist, und

$R^4$Hydroxy, $C_1$-$C_5$-Alkoxy, gegebenenfalls substituiertes Phenoxy, gegebenenfalls substituiertes Phenylsulfonyl, Pyrrolidino, Piperidino, Morpholino oder den Rest einer C-H-aciden Verbindung bedeuten und in der

der Ring A durch einen Benzoring anelliert, durch $C_1$-$C_4$-Alkyl, Chlor oder Brom substituiert oder in Ringposition 7 durch $C_1$-$C_5$-Mono- oder Dialkylamino, das gegebenenfalls jeweils wiederum durch Chlor oder Phenyl substituiert ist, Pyrrolidino, Piperidino, Morpholino, Hydroxy oder $C_1$-$C_4$-Alkoxy substituiert sein kann.

Alle in den obengenannten Resten auftretenden Alkylgruppen können sowohl geradkettig als auch verzweigt sein.

$R^1$ in Formel I bedeutet z.B. Methyl, Ethyl, Propyl, Isopropyl, Butyl, Isobutyl, tert-Butyl, Pentyl, Isoamyl, Hexyl, Heptyl, Octyl, 2-Ethylhexyl; Phenyl, durch $C_1$-$C_4$-Alkyl, $C_1$-$C_4$-Alkoxy oder Halogen substituiertes Phenyl, wie 4-Methylphenyl, 2-Methylphenyl, 4-Ethylphenyl, 4-Isopropylphenyl, 4-Methoxyphenyl, 2-Ethoxyphenyl, 4-Chlorphenyl, 2,4-Dichlorphenyl; Methoxy, Ethoxy, Propoxy, Isopropoxy, Butoxy; Fluor, Chlor oder Brom.

$R^2$ in Formel I bedeutet z.B. Wasserstoff oder zusammen mit $R^1$ 1,2-Ethylen, 1,2-Propylen oder 1,3-Propylen.

$R^3$ in Formel I bedeutet z.B. Phenyl oder vorzugsweise in Ringposition 4 durch Methyl, Ethyl, Propyl, Isopropyl, Butyl, sec-Butyl, Benzyl, 2-Phenylethyl, Cyclohexyl, Fluor, Chlor, Brom, Methoxy, Ethoxy, Propoxy, Isopropoxy, Butoxy, Pentyloxy, Hexyloxy, 2-Ethylhexyloxy, Methylamino, Ethylamino, Isopropylamino, Butylamino, 2-Ethylhexylamino, Benzylamino, 2-Chlorethylamino, Dimethylamino, Diethylamino, Dibutylamino, Methylethylamino, Di(2-ethylhexyl)amino, Anilino, 4-Methylanilino, 4-Ethylanilino, N-Methylanilino, N-Ethylanilino, Pyrrolidino, Piperidino oder Morpholino substituiertes Phenyl-; 2,4-Dimethylphenyl, 2-Methoxycarbonyl-4-dimethylaminophenyl, 2-Ethoxycarbonyl-4-dimethylaminophenyl, 2-Dimethylaminocarbonyl-4-methoxyphenyl, 2-Pyrrolidinocarbonyl-4-methylphenyl, 2-Piperidinocarbonyl-4-dimethylaminophenyl, 2-Morpholinocarbonyl-4-methylphenyl, 2,4-Dimethoxyphenyl, 3,4-Dimethoxyphenyl, 2-Methoxy-4-dimethylaminophenyl, 2-Methoxy-4-dibenzylaminophenyl oder 2,4-Dichlorphenyl; Naphthyl, 4-Methoxynaphthyl; Carbazol-3-yl, N-Methylcarbazol-3-yl, N-Ethylcarbazol-3-yl, N-Benzylcarbazol-3-yl, 1,2,3,4,4a,9a-Hexahydrocarbazol-6-yl, N-Methyl-1,2,3,4,4a,9a-hexahydrocarbazol-6-yl, N-Ethyl-1,2,3,4,4a,9a-hexahydrocarbazol-6-yl, N-Benzyl- 1,2,3,4,4a,9a-hexahydrocarbazol-6-yl; Indol-3-yl, 1-Methylindol-3-yl, 1-Ethylindol-3-yl, 1-Benzylindol-3-yl, 1-(2-Phenylethyl)indol-3-yl, 2-Methylindol-3-yl, 1,2-Dimethylindol-3-yl, 1-Benzyl-2-methylindol-3-yl; 2-Methylaminothiazol-5-yl, 2-Dimethylaminothiazol-5-yl, 2-Diethylaminothiazol-5-yl, 2-Benzylaminothiazol-5-yl, 2-Pyrrolidinothiazol-5-yl, 2-Morpholinothiazol-5-yl, 4-Methyl-2-dimethylaminothiazol-5-yl, 4-Phenyl-2-benzylaminothiazol-5-yl oder 4-Chlor-2-piperidinothiazol-5-yl.

$R^4$ in Formel I bedeutet z.B. Hydroxy; Methoxy, Ethoxy, Propoxy, Isopropoxy, sec-Butoxy, Pentyloxy; Phenoxy, durch $C_1$-$C_4$-Alkyl, $C_1$-$C_4$-Alkoxy oder Halogen substituiertes Phenoxy, wie 2-Methylphenoxy, 4-Methylphenoxy, 3-Methoxyphenoxy, 4-Chlorphenoxy; Phenylsulfonyl, durch $C_1$-$C_4$-Alkyl oder Halogen substituiertes Phenylsulfonyl, wie 4-Methylphenylsulfonyl, 4-Chlorphenylsulfonyl; Pyrrolidino, Piperidino oder Morpholino.

$R^4$ in Formel I bedeutet weiterhin den Rest einer C-H-aciden Verbindung. Reste von C-H-aciden Verbindungen sind beispielsweise 2-(Pyrrolidino-, Piperidino- oder Morpholino-)cyclopent-1-en-1-yl oder -cyclohex-1-en-1-yl; gegebenenfalls in Ringposition 5 durch $C_1$-$C_4$-Alkyl mono-oder disubstituiertes Cyclohexan-1,3-dion-2-yl, wie Cyclohexan-1,3-dion-2-yl, 5-Methylcyclohexan-1,3-dion-2-yl, 5-Ethylcyclohexan-1,3-dion-2-yl oder 5,5-Dimethylcyclohexan-1,3-dion-2-yl; Benzoylmethyl; Cyano; Nitromethyl; 2,4,6-Trihydroxypyrimid-5-yl; 1-Phenyl-3-methylpyrazol-5-on-4-yl; 5-Hydroxy-3,4-dichlorfuran-2-yloxy; oder der Rest

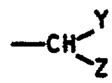

in dem Y und Z gleich oder verschieden sind und jeweils unabhängig voneinander für Acetyl, Benzoyl, $C_1$-$C_5$-Alkoxycarbonyl oder Cyano stehen und in dem für den Fall, daß Y die Bedeutung von Cyano besitzt, Z auch für Methyl stehen kann, wie Bis(acetyl)methyl, Bis(benzoyl)methyl, Bis(methoxycarbonyl)methyl, Bis(ethoxycarbonyl)methyl, Bis(cyano)methyl, Acetyl-benzoyl-methyl, Acetyl-methoxycarbonyl-methyl, Benzoyl-ethoxycarbonyl-methyl, Cyano-methoxycarbonyl-methyl, Cyanoethoxycarbonyl-methyl oder 1-Cyanoeth-1-yl.

Der Ring A in Formel I kann beispielsweise in Ringposition 6 oder 8 durch Chlor oder Brom oder in Ringposition 6 und 8 durch Chlor substituiert sein. Er kann weiterhin in Ringposition 7 beispielsweise Methyl, Ethyl, Propyl, Isopropyl, Butyl; Hydroxy; Methoxy, Ethoxy, Isopropoxy; Methylamino, Ethylamino, Propylamino, Isopropylamino, Butylamino, Benzylamino, 2-Chlorethylamino, Dimethylamino, Diethylamino, Dibutylamino, Dibenzylamino, Methyl-ethyl-amino; Pyrrolidino, Piperidino oder Morpholino als Substituenten aufweisen. Außerdem kann er z.B. wie folgt benzoanelliert sein:

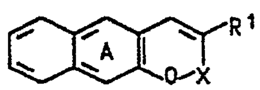 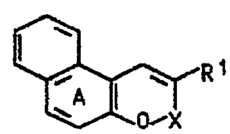 oder 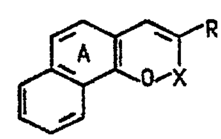

Bevorzugt sind solche Benzopyrane der Formel I, in der $R^1$ $C_1$-$C_5$-Alkyl, $R^2$ Wasserstoff, $R^3$ Carbazol-3-yl oder gegebenenfalls substituiertes Phenyl und $R^4$ den Rest

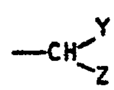

bedeuten.

Die erfindungsgemäßen Benzopyrane werden vorteilhaft erhalten, wenn man eine Benzopyryliumverbindung der Formel II

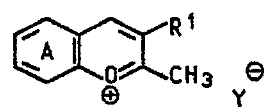 (II)

in der $R^1$ und der Ring A die obengenannte Bedeutung besitzen und $Y^\ominus$ ein Anion bedeutet, mit einem Aldehyd der Formel III

$R^3$ - CHO (III),

in der $R^3$ die obengenannte Bedeutung besitzt, im Molverhältnis 1 : 0,8 bis 1 : 1,2 in Gegenwart eines inerten Lösungsmittels bei einer Temperatur von 20 bis 120°C, vorzugsweise 40 bis 80°C, umsetzt und in einer Folgestufe das resultierende Farbsalz der Formel IV

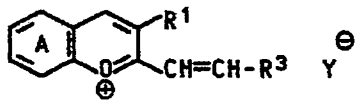

(IV)

in der $R^1$, $R^3$, $Y^\ominus$ und der Ring A die obengenannte Bedeutung besitzen, mit einer Verbindung der Formel V

$R^4$ - H (V),

in der $R^4$ die obengenannte Bedeutung besitzt, im Molverhältnis 1 : 1,1 bis 1 : 2 in Gegenwart eines inerten Lösungsmittels und einer Base bei einer Temperatur von 20 bis 120°C, vorzugsweise 40 bis 80°C, zur Reaktion bringt.

Geeignete Anionen $Y^\ominus$ sind z.B. Trichlorozinkat, Tetrachloroferrat(III), Hydrogensulfat, Nitrat oder Halogenid, wie Chlorid oder Bromid. Besonders bevorzugt sind Trichlorozinkat und Tetrachloroferrat(III).

Als inerte Lösungsmittel verwendet man zweckmäßig Alkohole, z.B. Methanol, Ethanol, Propanol, Isopropanol, Butanol oder Isobutanol. Gegebenenfalls werden auch Mischungen dieser Alkohole mit aromatischen Kohlenwasserstoffen, wie Toluol oder Xylol als Reaktionsmedium eingesetzt.

Geeignete Basen für die Umsetzung des Farbsalzes IV mit der Verbindung V sind beispielsweise Alkali- oder Erdalkalicarbonate, wie Natriumcarbonat, Kaliumcarbonat, Magnesiumcarbonat oder Calciumcarbonat; Erdalkalioxide, wie Magnesiumoxid oder Calciumoxid; oder Alkalialkanolate, wie Natrium- oder Kaliummethanolat, -ethanolat oder -butanolat. Im allgemeinen gibt man 1 bis 3 Mol Base, bezogen auf ein Mol Farbsalz, zu.

Die für das erfindungsgemäße Verfahren benötigten Benzopyryliumsalze II, Aldehyde III und Verbindungen IV sind bekannt.

Die erfindungsgemäßen Benzopyrane sind schwach farbige bis farblose Verbindungen, deren Lösungen in inerten organischen Lösungsmitteln im Kontakt mit Elektronenacceptoren, je nach der Substitution des Benzopyrans, Färbungen in gelben, orangen, roten und blauen Farbtönen geben. Beispiele für Elektronenacceptorsubstanzen sind Carbon- oder Mineralsäuren, Kaolin, Bentonit, aktivierter Ton, Aluminiumsilikat, Attapulgit oder jeder beliebige Ton, sauer reagierende polymere Materialien, wie Kondensationsprodukte auf der Basis Phenol (und/oder Phenolsulfonsäuren) und Formaldehyd, ferner Metalloxide oder -salze, wie Zinkoxid, Aluminiumoxid, Zinkchlorid, Eisenstearat oder Cobaltnaphthenat.

Aufgrund dieser Eigenschaften sind die neuen Verbindungen der Formel I als Farbbildner zur Verwendung in druck- und wärmeempfindlichen Aufzeichnungsmaterialien geeignet.

Für die Anwendung in druckempfindlichen Systemen werden die neuen Benzopyrane vorteilhaft in Form von Lösungen in organischen Lösungsmitteln, z.B. Chlorparaffine, partiell hydriertes Di- oder Terphenyl, Alkylbenzole, Alkylnaphthaline, alkylierte Dibenzylbenzole, Paraffinöl, Mineralöl oder auch übliche tiefer siedende Lösungsmittel, wie Xylol oder Toluol, in Mikrokapseln eingeschlossen und mit diesen der Träger, z.B. Papier beschichtet. Bei Druck tritt dann im Kontakt mit Elektronenacceptoren an der Druckstelle Farbbildung ein.

Geeignete Verfahren zur Herstellung von Mikrokapseln sind z.B. aus der US-A-2 800 457, US-A-2 800 458, DE-A-2 119 933 und EP-A-26 914 bekannt. Man kann die erfindungsgemäßen Verbindungen auch nach dem in der US-A-3 103 404 beschriebenen Verfahren in Wachs oder Öl-Wachsmischungen fein verteilen und mit diesen Mischungen Träger, wie Folien oder Papier beschichten. Man erhält druckempfindliche Materialien, die zum Durchschreiben auf mit Elektronenacceptorsubstanzen beschichteten Papieren geeignet sind und die nach Gebrauch wie Kohlepapier entfernt werden.

Die erfindungsgemäßen Benzopyrane können auch als Farbbildner in wärmeempfindlichen Aufzeichnungsmaterialien verwendet werden, die auf einem Träger ein Bindemittel, einen Farbbildner und eine Elektronenacceptorsubstanz enthalten. Der Aufbau solcher wärmeempfindlicher Aufzeichnungsmaterialien und die Zusammensetzung der durch Wärmeeinfluß die Farbe erzeugenden Schichten sind bekannt (z.B. DE-A-2 228 581, DE-A-2 110 854), ebenso die Verfahren und Vorrichtungen, mit denen die Farbbildung erreicht wird.

Die folgenden Beispiele sollen die Erfindung näher erläutern.

## Beispiel 1

a) Synthese des Farbsalzes

331 g (1 Mol) 2,3-Dimethylbenzopyrylium-trichlorozinkat und 149 g (1 Mol) 4-Dimethylaminobenzaldehyd wurden in 1500 ml Methanol 4,5 Stunden lang unter Rückfluß erhitzt. Nach dem Abkühlen wurde der abgeschiedene Farbstoff abgesaugt und mit Methanol gewaschen. Ausbeute: 400 g (86,6 % d. Th.) Fp: 242-244°C

Man erhielt ein Farbsalz der Formel

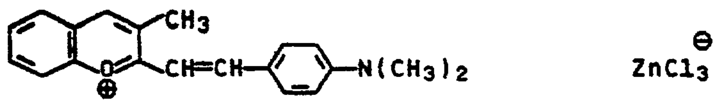

b) Synthese des Farbbildners

46 g (0,1 Mol) des unter a) hergestellten Farbsalzes und 20 g Benzoylaceton (0,13 Mol) wurden in 500 ml Methanol in Gegenwart von 22 g (0,2 Mol) Natriumcarbonat bei einer Temperatur von 45°C gerührt. Das Reaktionsgemisch wurde dann in ein Gemisch aus 1600 ml Toluol und 1600 ml Wasser gegeben. Die organische Phase wurde abgetrennt, mit Aktivkohle behandelt, filtriert und eingeengt. Nach Zugabe von 500 ml Methanol bildete sich ein Niederschlag, der abgesaugt, mit 200 ml Methanol gewaschen und bei einer Temperatur von 60°C unter vermindertem Druck getrocknet wurde. Ausbeute: 34 g (76 %) Fp: 165-167°C

Man erhielt einen Farbbildner der Formel

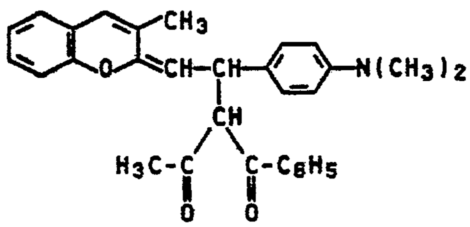

Die in Tabelle 1 aufgeführten Komponenten der Formel

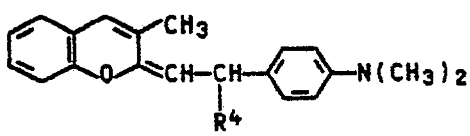

können in analoger Weise erhalten werden.

**Tabelle 1**

| Beispiel | $R^4$ | Fp [°C] | Farbe bzw. $\lambda_{max}$ [nm] |
|---|---|---|---|
| 2 | 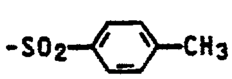 | 270-272 | blau |
| 3 | $-CH(CO-C_6H_5)_2$ | 193-195 | 628,9 |
| 4 | $-CH(CN)_2$ | 194-196 | 628,9 |
| 5 | $-CH_2-NO_2$ | 156-158 | blau |

Beispiel 6

a) Synthese des Farbsalzes

331 g (1 Mol) 2,3-Dimethylbenzopyryliumtrichlorozinkat und 223 g (1 Mol) N-Ethylcarbazol-3-aldehyd wurden in 2600 ml mit HCl gesättigtem Methanol 3 Stunden lang unter Rückfluß erhitzt. Nach dem Abkühlen wurde der abgeschiedene Farbstoff abgesaugt und mit Methanol gewaschen. Ausbeute: 453 g (84,6 % d. Th.) Fp: 240-242°C

Man erhielt ein Farbsalz der Formel

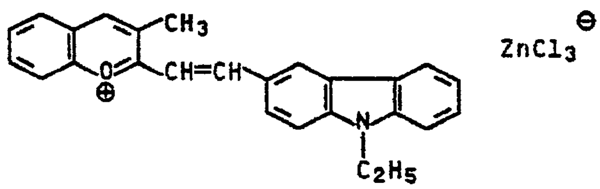

b) Synthese des Farbbildners

16 g (0,03 Mol) des unter a) hergestellten Farbsalzes und 5 g (0,038 Mol) Acetessigester wurden in 250 ml Methanol in Gegenwart von 7 g (0,066 Mol) Natriumcarbonat bei einer Temperatur von 45°C gerührt. Das Reaktionsgemisch wurde dann in ein Gemisch aus 500 ml Toluol und 500 ml Wasser gegeben. Die organische Phase wurde abgetrennt, mit Aktivkohle behandelt, filtriert und eingeengt. Nach Zugabe von 250 ml Methanol bildete sich ein Niederschlag, der abgesaugt, mit 70 ml Methanol gewaschen und bei einer Temperatur von 60°C unter vermindertem Druck getrocknet wurde. Ausbeute: 10 g (67,6 % d. Th.) Fp: 132-134°C

Man erhielt einen Farbbildner der Formel

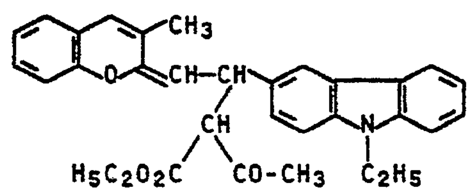

Die in Tabelle 2 aufgeführten Komponenten der Formel

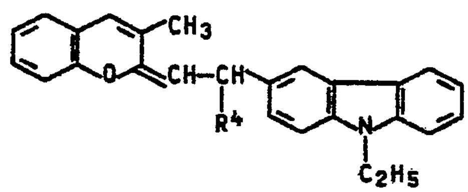

können in analoger Weise erhalten werden.

**Tabelle 2**

| Beispiel | $R^4$ | Fp [°C] | Farbe bzw. $\lambda_{max}$[nm] |
|---|---|---|---|
| 7 | 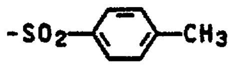 | 156-158 | blau |
| 8 | $-CH(CO-CH_3)_2$ | 147-149 | 607,4 |
| 9 | 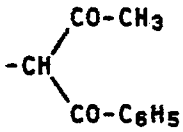 | 126-128 | 606,9 |
| 10 | $-OH$ | 154-157 | 606,4 |
| 11 | $-CH(CO-OC_2H_5)_2$ | 182-184 | hellblau |
| 12 | $-CH(CO-C_6H_5)_2$ | 142-144 | 606,9 |
| 13 | $-CH(CN)_2$ | 241-243 | 607,4 |
| 14 | 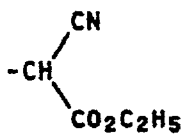 | 193-195 | 607,9 |
| 15 | 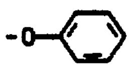 | 187-189 | 607,9 |
| 16 | $-CH_2-NO_2$ | 154-156 | 605,4 |
| 17 | $-CH(CO-OCH_3)_2$ | | |

Analog den Beispielen 1 und 6 können die in Tabelle 3 aufgeführten Farbbildner der Formel

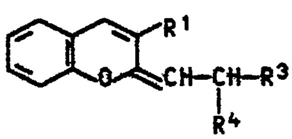

erhalten werden.

Tabelle 3

| Beispiel | $R^1$ | $R^3$ | $R^4$ | Fp [°C] | Farbe bzw. $\lambda_{max}$[nm] |
|---|---|---|---|---|---|
| 18 | $-CH_3$ | 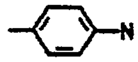 $-N(CH_2-C_6H_5)_2$ | $-SO_2-$ 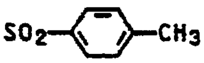 $-CH_3$ | 146-148 | blau |
| 19 | $-CH_3$ | 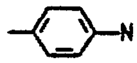 $-N(CH_2-C_6H_5)_2$ | $-CH(CO-CH_3)_2$ | 142-144 | lindgrün |
| 20 | $-CH_3$ | 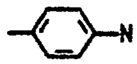 $-N(CH_2-C_6H_5)_2$ | $-CH(CO-C_6H_5)_2$ | 144-146 | 642,4 |
| 21 | $-CH_3$ | 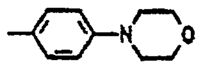 | $-SO_2-$ 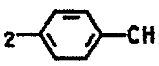 $-CH_3$ | 203-205 | blau |
| 22 | $-CH_3$ | 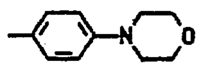 | $-CH(CO-CH_3)_2$ | 170-172 | blau |
| 23 | $-CH_3$ | 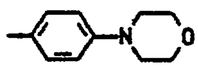 | 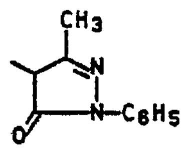 | 144-148 | grün |
| 24 | $-CH_3$ | 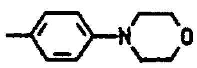 | $-CH(CO-C_6H_5)_2$ | 189 | 632,4 |
| 25 | $-CH_3$ | 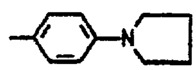 | $-SO_2-$ 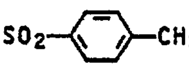 $-CH_3$ | 184-186 | blau |
| 26 | $-CH_3$ | 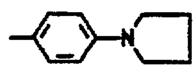 | 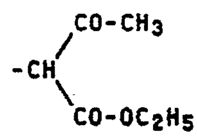 | Öl | 672,9/631,4 |
| 27 | $-CH_3$ | 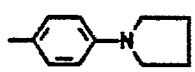 | 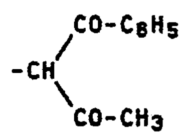 | 128-130 | 671,9/630.9 |
| 28 | $-CH_3$ | 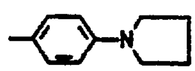 | $-CH(CO-C_6H_5)_2$ | 132-134 | 672,9/630,4 |
| 29 | $-CH_3$ | 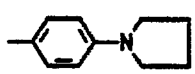 | $-CH(CN)_2$ | 203-205 | blau |
| 30 | $-CH_3$ | 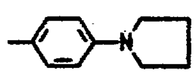 | 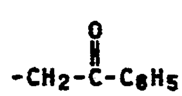 | Öl | blau |
| 31 | $-CH_3$ | 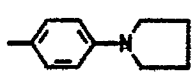 | 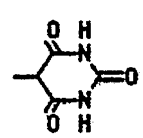 | Öl | blau |
| 32 | $-CH_3$ | 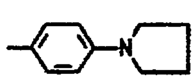 | 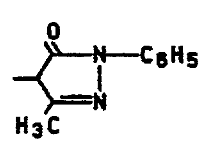 | 154-156 | blau |

Tabelle 3 (Fortsetzung)

| Beispiel | $R^1$ | $R^3$ | $R^4$ | Fp [°C] | Farbe bzw. $\lambda_{max}$[nm] |
|---|---|---|---|---|---|
| 33 | $-CH_3$ | 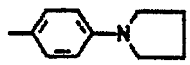 | -OH | 195 | blau |
| 34 | $-CH_3$ | 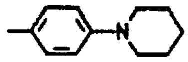 | $-CH(CO-C_6H_5)_2$ | 178-180 | 519,3 |
| 35 | $-CH_3$ | 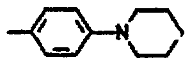 | $-CH(CO-CH_3)_2$ | | |
| 36 | $-CH_3$ | 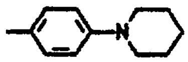 | 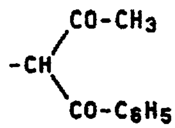 | | |
| 37 | $-CH_3$ | 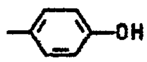 | $-CH(CO-CH_3)_2$ | 165 | rotviolett |
| 38 | $-CH_3$ | 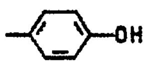 | 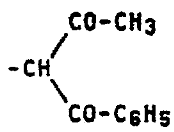 | | rotviolett |
| 39 | $-CH_3$ | 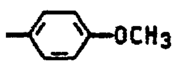 | $-CH(CO-CH_3)_2$ | 98-102 | 523,8 |
| 40 | $-CH_3$ | 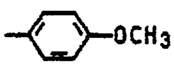 | 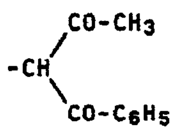 | 159-160 | rotviolett |
| 41 | $-CH_3$ | 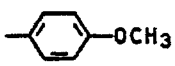 | -OH | 136-140 | hellrot |
| 42 | $-CH_3$ | 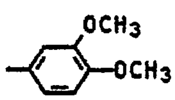 | 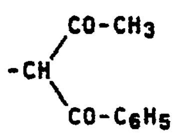 | Öl | 542,8 |
| 43 | $-CH_3$ | 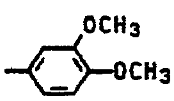 | -OH | 120-124 | rotviolett |
| 44 | $-CH_3$ | 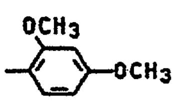 | $-CH(CO-CH_3)_2$ | | violett |
| 45 | $-CH_3$ | 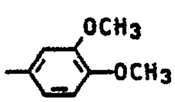 | -OH | 210-216 | violett |
| 46 | $-CH_3$ | 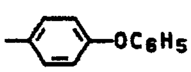 | $-CH(CO-CH_3)_2$ | 141-145 | rosa |
| 47 | $-CH_3$ | 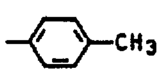 | $-CH(CO-CH_3)_2$ | Öl | orange |

Tabelle 3 (Fortsetzung)

| Beispiel | $R^1$ | $R^3$ | $R^4$ | Fp [°C] | Farbe bzw. $\lambda_{max}$[nm] |
|---|---|---|---|---|---|
| 48 | $-CH_3$ | 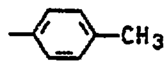 | -OH | Öl | orange |
| 49 | $-CH_3$ | 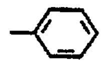 | $-CH(CO-CH_3)_2$ | 118 | gelb |
| 50 | $-CH_3$ | 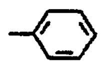 | 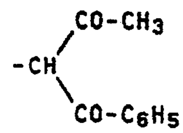 | 155 | gelb |
| 51 | $-CH_3$ | 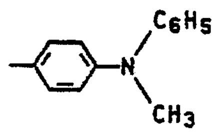 | 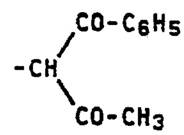 | 163-168 | 647,9 |
| 52 | $-CH_3$ | 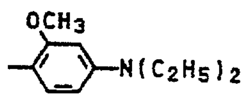 | $-CH(CO-CH_3)_2$ | Öl | blau |
| 53 | $-CH_3$ | 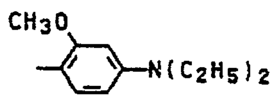 | 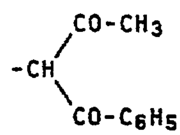 | Öl | blau |
| 54 | $-CH_3$ | 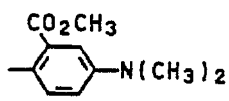 | 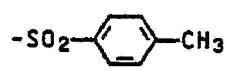 | 181-183 | blau |
| 55 | $-CH_3$ | 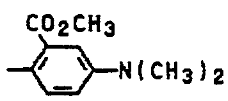 | $-CH(CO-CH_3)_2$ | 130-132 | blau |
| 56 | $-CH_3$ | 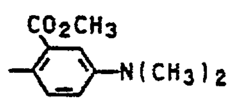 | $-CH(CO-C_6H_5)_2$ | 166-168 | 644,9 |
| 57 | $-CH_3$ | 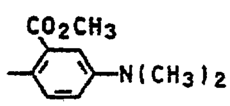 | $-CH(CN)_2$ | 221-223 | 643,9 |
| 58 | $-CH_3$ | 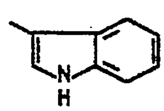 | $-CH(CO-CH_3)_2$ | 198-200 | 573,3 |
| 59 | $-CH_3$ | 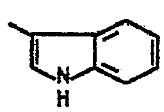 | 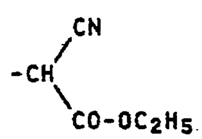 | 134-137 | violett |

<u>Tabelle 3</u> (Fortsetzung)

| Beispiel | $R^1$ | $R^3$ | $R^4$ | Fp [°C] | Farbe bzw. $\lambda_{max}$[nm] |
|---|---|---|---|---|---|
| 60 | $-CH_3$ | 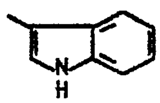 | 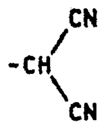 | | blau |
| 61 | $-CH_3$ | 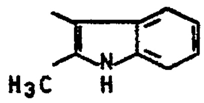 | 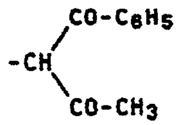 | 204-207 | violett 549,3/583,8 |
| 62 | $-CH_3$ | 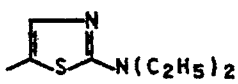 | 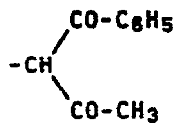 | Öl | blau |
| 63 | $-CH_3$ | 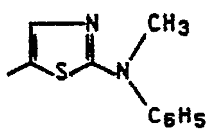 | 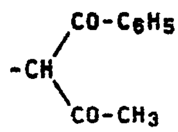 | Öl | blau |
| 64 | $-CH_3$ | 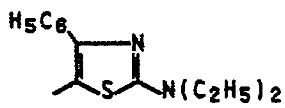 | 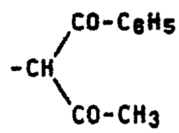 | Öl | blau |
| 65 | $-CH_3$ | 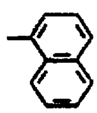 | -OH | 215 | violett |
| 66 | $-C_2H_5$ | 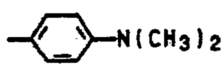 | 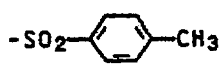 | 148-150 | blau |
| 67 | $-C_2H_5$ | 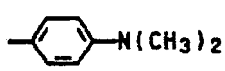 | $-CH(CO-CH_3)_2$ | 128-130 | blau |
| 68 | $-C_2H_5$ | 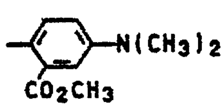 | 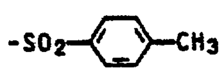 | 166-168 | blau |
| 69 | $-C_2H_5$ | 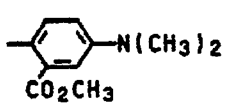 | $-CH(CO-CH_3)_2$ | 131-135 | blau |
| 70 | $-CH(CH_3)_2$ | 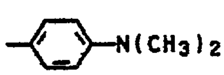 | 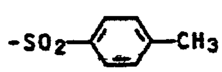 | 157-159 | blau |
| 71 | $-CH(CH_3)_2$ | 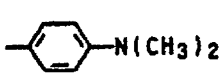 | $-CH(CO-CH_3)_2$ | 133-135 | blau |

Tabelle 3 (Fortsetzung)

| Beispiel | $R^1$ | $R^3$ | $R^4$ | Fp [°C] | Farbe bzw. $\lambda_{max}$[nm] |
|---|---|---|---|---|---|
| 72 | $-CH(CH_3)_2$ | 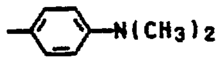 | 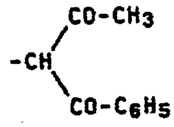 | 153-155 | 633,4 |
| 73 | $-CH(CH_3)_2$ | 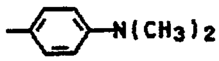 | $-CH(CN)_2$ | 195-197 | 633,4 |
| 74 | $-CH(CH_3)_2$ | 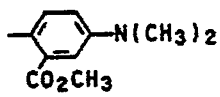 | 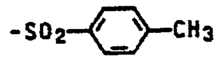 | 129-131 | himmelblau |
| 75 | $-CH(CH_3)_2$ | 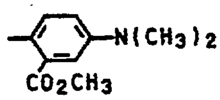 | $-CH(CO-CH_3)_2$ | 148-150 | himmelblau |
| 76 | $-CH(CH_3)_2$ | 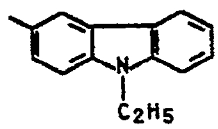 | $-CH(CO-CH_3)_2$ | 164-168 | blau |
| 77 | $-CH(CH_3)_2$ | 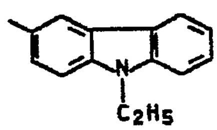 | 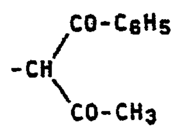 | 118-120 | 605,4 |
| 78 | $-CH(CH_3)_2$ | 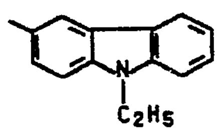 | $-CH(CO-C_6H_5)_2$ | 143-145 | 606,4 |
| 79 | $-CH(CH_3)_2$ | 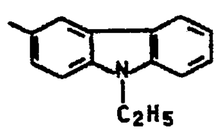 | 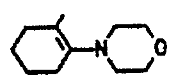 | 141-143 | hellblau |
| 80 | $-CH_2-CH(CH_3)_2$ | 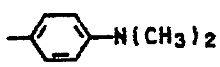 | 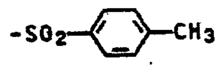 | 140-142 | blau |
| 81 | $-CH_2-CH(CH_3)_2$ | 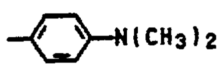 | $-CH(CO-CH_3)_2$ | Öl | blau |
| 82 | $-CH_2-CH(CH_3)_2$ | 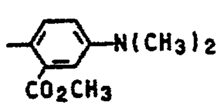 | 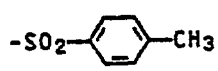 | 163-165 | hellblau |
| 83 | $-CH_2-CH(CH_3)_2$ | 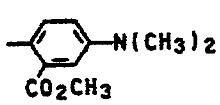 | $-CH(CO-CH_3)_2$ | Öl | grünblau |
| 84 | $-CH_2-CH(CH_3)_2$ | 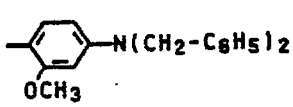 | $-CH(CO-CH_3)_2$ | Öl | blau bis blaugrün |

<u>Tabelle 3</u> (Fortsetzung)

| Beispiel | $R^1$ | $R^3$ | $R^4$ | Fp [°C] | Farbe bzw. $\lambda_{max}$[nm] |
|---|---|---|---|---|---|
| 85 | 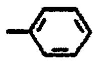 | 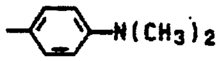 | $-CH(CO-CH_3)_2$ | Öl | blau |
| 86 | 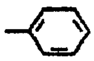 | 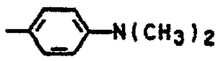 | 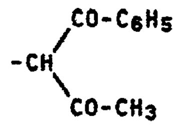 | Öl | blau |
| 87 | 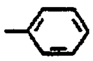 | 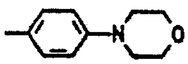 | $-CH(CO-CH_3)_2$ | Öl | blau |
| 88 | 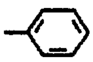 | 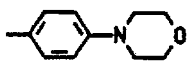 | 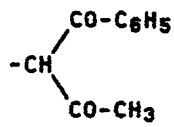 | Öl | blau |
| 89 | 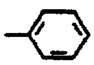 | 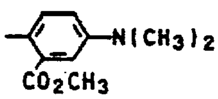 | 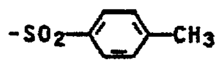 | 179-181 | türkisblau |
| 90 | 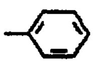 | 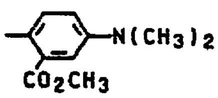 | $-CH(CO-CH_3)_2$ | Öl | grünblau |
| 91 | 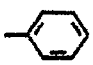 | 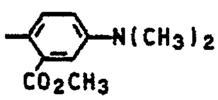 | $-OCH_3$ | | grünblau |
| 92 | 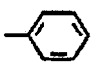 | 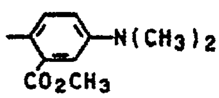 | 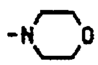 | Öl | grünblau |
| 93 | 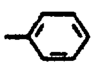 | 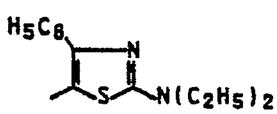 | 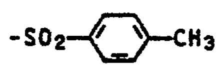 | 180-182 | blau |
| 94 | 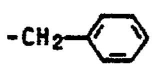 | 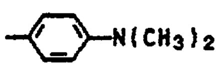 | 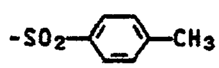 | 198-200 | blau |
| 95 | 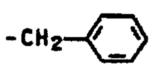 | 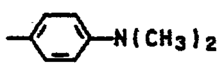 | $-CH(CO-CH_3)_2$ | 162-164 | blau |
| 96 | $-OCH_3$ | 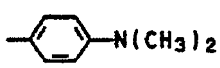 | 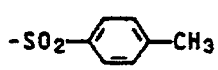 | 182 | blau |
| 97 | $-OCH_3$ | 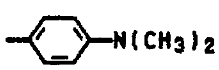 | $-CH(CO-CH_3)_2$ | 168-170 | blau |
| 98 | $-OCH_3$ | 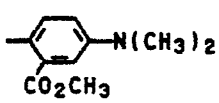 | 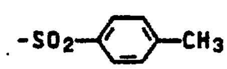 | 100-112 | blau |

Tabelle 3 (Fortsetzung)

| Beispiel | $R^1$ | $R^3$ | $R^4$ | Fp [°C] | Farbe bzw. $\lambda_{max}$[nm] |
|---|---|---|---|---|---|
| 99 | $-OCH_3$ | 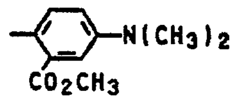 | $-CH(CO-CH_3)_2$ | Öl | blau |
| 100 | $-OCH_3$ | 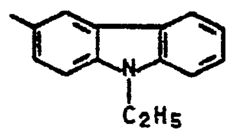 | $-CH(CO-CH_3)_2$ | 183-185 | blau |

Beispiel 101

Analog Beispiel 1 wurde der folgende Farbbildner erhalten:

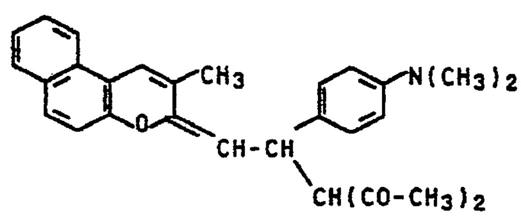

Fp: 183-185°C

Beispiel 102

a) Synthese des Farbsalzes

24 g (0,1 Mol) Bis(4-formylphenyl)methylamin und 66 g (0,2 Mol) 2,3-Di-methylbenzopyrylium-trichlorozinkat wurden in 200 ml Methanol 3 Stunden und nach Zugabe von 200 ml Methylglykol 1/2 Stunde lang unter Rückfluß erhitzt. Nach dem Abkühlen wurde das Lösungsmittel vom abgeschiedenen Farbstoff abgetrennt. Ausbeute: 66 g Fp: 110°C

b) Synthese des Farbbildners

26 g (0,03 Mol) des unter a) hergestellten Farbsalzes und 8 g (0,08 Mol) Acetylaceton wurden in Gegenwart von 14 g (0,13 Mol) Natriumcarbonat in einem Gemisch aus 200 ml Methanol und 100 ml Toluol 3 Stunden auf 50°C erwärmt. Das Reaktionsgemisch wurde dann in ein Gemisch aus 500 ml Toluol und 500 ml Wasser gegeben. Die organische Phase wurde abgetrennt, mit Aktivkohle behandelt, filtriert und eingeengt. Der Rückstand wurde in eisgekühltes Methanol getropft und der dabei gebildete Niederschlag bei 0°C abgesaugt. Ausbeute: 7 g Fp: 166-168°C

Man erhielt einen Farbbildner der Formel

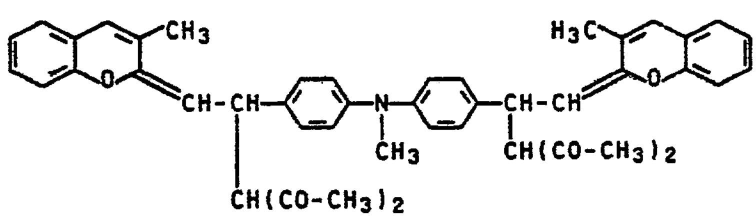

Beispiel 103

Analog Beispiel 102 b wurde mit dem in Beispiel 102 a hergestellten Farbsalz der folgende Farbbildner hergestellt:

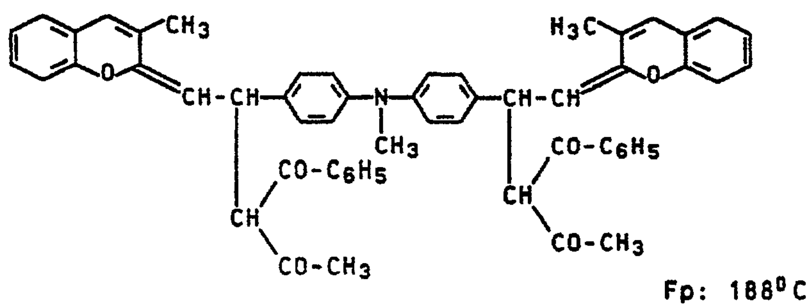

Fp: 188°C

Beispiel 104

a) Synthese des Farbsalzes

66g (0,2Mol) 2,3-Dimethylbenzopyrylium-trichlorozinkat und 33 g (0,1Mol) Ethylen-bis[N-ethyl-N-(4-formylphenyl)]-amin wurden in 400 ml Methanol 4 Stunden lang unter Rückfluß erhitzt. Nach dem Abkühlen wurde der abgeschiedene Farbstoff abgesaugt und mit Methanol gewaschen. Ausbeute: 82g Fp: 255-260C

b) Synthese des Farbbildners

28,5 g (0,03 Mol) des unter a) hergestellten Farbsalzes und 13 g (0,08 Mol) Benzoylaceton wurden in Gegenwart von 15 g (0,14 Mol) Natriumcarbonat in einem Gemisch aus 350 ml Methanol und 100 ml Toluol 7 Stunden lang unter Rückfluß gerührt. Das Reaktionsgemisch wurde dann in ein Gemisch aus 500 ml Toluol und 500 ml Wasser gegeben. Die organische Phase wurde abgetrennt, mit Aktivkohle behandelt, filtriert und eingeengt. Der Rückstand wurde mit Methylglykol verdünnt und mit Methanol versetzt. Der dabei gebildete Niederschlag wurde abgesaugt, mit Methanol gewaschen und bei einer Temperatur von 60°C unter vermindertem Druck getrocknet. Ausbeute: 7g Fp: 132-140°C

Man erhielt einen Farbbildner der Formel

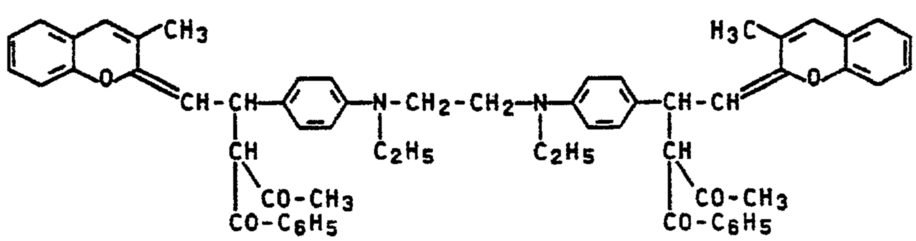

Beispiel 105

a) Synthese des Farbsalzes

21,9 g (0,05 Mol) 2-Cyclohexyliden-2,3,4,5-tetrahydroxanthylium-trichlorozinkat und 7,2 g (0,05 Mol) Indolyl-3-aldehyd wurden in 320 ml n-Butanol eine Stunde lang unter Rückfluß erhitzt. Der nach dem Abkühlen abgeschiedene Farbstoff wurde abgesaugt. Ausbeute: 21g Fp: 243-245°C

Man erhielt ein Farbsalz der Formel

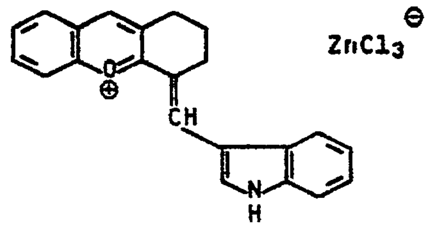

b) Synthese des Farbbildners

9,7 g (0,02 Mol) des unter a) hergestellten Farbsalzes und 2.7 g (0,027 Mol) Acetylaceton wurden in 100 ml Methanol in Gegenwart von 4.7 g (0,044 Mol) Natriumcarbonat 4 Stunden lang bei einer Temperatur von 45°C gerührt. Das Reaktionsgemisch wurde dann in ein Gemisch aus 300 ml Toluol und 300 ml Wasser gegeben. Die organische Phase wurde abgetrennt, mit Aktivkohle behandelt, filtriert und eingeengt. Nach Zugabe von 100 ml Methanol bildete sich ein Niederschlag, der abgesaugt, mit 40 ml Methanol gewaschen und bei einer Temperatur von 60°C unter vermindertem Druck getrocknet wurde. Ausbeute: 5 g Fp: 162-170°C

Man erhielt einen Farbbildner der Formel

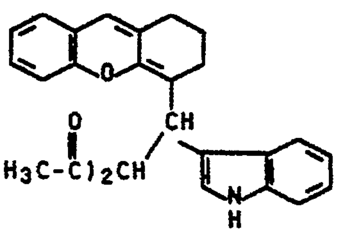

## Patentansprüche

1. Benzopyrane der Formel I

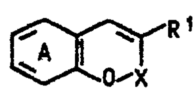

(I),

in der
$R^1$ für gegebenenfalls substituiertes $C_1$-$C_8$-Alkyl, gegebenenfalls substituiertes Phenyl, $C_1$-$C_5$-Alkoxy oder Halogen und
X für die Reste

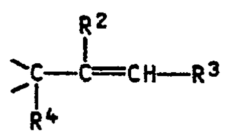 oder 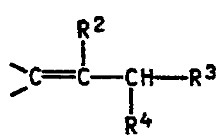 stehen, wobei

$R^2$ Wasserstoff oder zusammen mit $R^1$ gegebenenfalls durch $C_1$-$C_4$-Alkyl substituiertes $C_2$-$C_3$-Alkylen,
$R^3$ Phenyl, das gegebenenfalls durch $C_1$-$C_4$-Alkyl, das wiederum durch Phenyl substituiert sein kann, Cyclohexyl, Halogen, $C_1$-$C_8$-Alkoxy, $C_1$-$C_8$-Mono- oder Dialkylamino, das jeweils wiederum durch Chlor oder Phenyl substituiert sein kann, Phenylamino, das wiederum durch $C_1$-$C_8$-Alkyl am Phenylring oder am Stickstoffatom substituiert sein kann, Pyrrolidino, Piperidino oder Morpholino substituiert ist, und das gegebenenfalls weiterhin durch $C_1$-$C_5$-Alkyl, $C_1$-$C_5$-Alkoxycarbonyl, $C_1$-$C_5$-Dialkylaminocarbonyl, Pyrrolidinocarbonyl, Piperidinocarbonyl, Morpholinocarbonyl, $C_1$-$C_8$-Alkoxy oder Halogen substituiert ist; gegebenenfalls durch $C_1$-$C_4$-Alkoxy substituiertes Naphthyl; Carbazol-3-yl oder 1,2,3,4,4a,9a-Hexahydrocarbazol-6-yl, das jeweils durch $C_1$-$C_5$-Alkyl oder Benzyl am Stickstoffatom substituiert sein kann; Indol-3-yl, das in Ringposition 1 und/oder 2 durch gegebenenfalls phenylsubstituiertes $C_1$-$C_5$-Alkyl substituiert sein kann; oder Thiazol-5-yl, das in Ringposition 2 durch gegebenenfalls phenylsubstituiertes $C_1$-$C_5$-Mono- oder Dialkylamino, Pyrrolidino, Piperidino oder Morpholino und in Ringposition 4 gegebenenfalls durch $C_1$-$C_5$-Alkyl, Phenyl oder Chlor substituiert ist, und

$R^4$Hydroxy, $C_1$-$C_5$-Alkoxy, gegebenenfalls substituiertes Phenoxy, gegebenenfalls substituiertes Phenylsulfonyl, Pyrrolidino, Piperidino, Morpholino oder den Rest einer C-H-aciden Verbindung bedeuten und in der
derRing A durch einen Benzoring anelliert, durch $C_1$-$C_4$-Alkyl, Chlor oder Brom substituiert oder in Ringposition 7 durch C1-$C_5$-Mono- oder Dialkylamino, das gegebenenfalls jeweils wiederum durch Chlor oder Phenyl substituiert ist, Pyrrolidino, Piperidino, Morpholino, Hydroxy oder $C_1$-$C_4$-Alkoxy substituiert sein kann.

2. Verfahren zur Herstellung von Benzopyranen der Formel I gemäß Anspruch 1, dadurch gekennzeichnet, daß man eine Benzopyryliumverbindung der Formel II

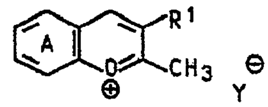

(II),

in der $R^1$ und der Ring A die in Anspruch 1 genannte Bedeutung besitzen und $Y^{\ominus}$ ein Anion bedeutet, mit einem Aldehyd der Formel III

$R^3$ - CHO (III),

in der $R^3$ die in Anspruch 1 genannte Bedeutung besitzt, in Gegenwart eines inerten Lösungsmittels bei einer Temperatur von 20 bis 120°C umsetzt und in einer Folgestufe das resultierende Farbsalz der Formel IV

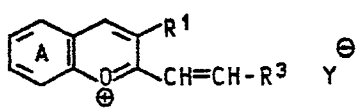

(IV),

in der $R^1$, $R^3$, $Y^{\ominus}$ und der Ring A die obengenannte Bedeutung besitzen, mit einer Verbindung der Formel V

$R^4$ - H (V),

in der $R^4$ die in Anspruch1 genannte Bedeutung besitzt, in einem inerten Lösungsmittel in Gegenwart einer Base bei einer Temperatur von 20 bis 120°C zur Reaktion bringt.

3. Verwendung der Benzopyrane der Formel I gemäß Anspruch 1 als Farbbildner in druck- oder wärmeempfindlichen Aufzeichnungssystemen.

## Claims

1. A benzopyran of the formula I

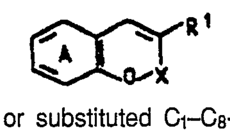

(I)

where $R^1$ is unsubstituted or substituted $C_1$–$C_8$-alkyl, unsubstituted or substituted phenyl, $C_1$–$C_5$-alkoxy or halogen, X is a radical

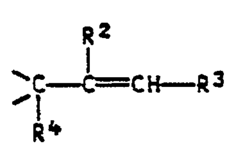

or

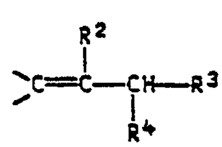

$R^2$ is hydrogen or, together with $R^1$, is $C_2$- or $C_3$-alkylene which is unsubstituted or substituted by $C_1$–$C_4$-alkyl,
$R^3$ is phenyl which is unsubstituted or substituted by $C_1$–$C_4$-alkyl (which in turn may be substituted by phenyl), cyclohexyl, halogen, $C_1$–$C_8$-alkoxy, $C_1$–$C_8$-mono- or dialkylamino (each of which in turn may be substituted by chlorine or phenyl), phenylamino (which in turn may be substituted by $C_1$–$C_8$-alkyl on the phenyl ring or on the nitrogen atom), pyrrolidino, piperidino or morpholino, and which furthermore may be substituted by $C_1$–$C_5$-alkyl, $C_1$–$C_5$-alkoxycarbonyl, $C_1$–$C_5$-dialkylaminocarbonyl, pyrrolidinocarbonyl, piperidinocarbonyl, morpholinocarbonyl, $C_1$–$C_8$-alkoxy or halogen; or is naphthyl which is unsubstituted or substituted by $C_1$–$C_4$-alkoxy; or is carbazol-3-yl or 1,2,3,4,4a,9a-hexahydrocarbazol-6-yl, each of

which may be substituted on the nitrogen atom by $C_1$–$C_5$-alkyl or benzyl; or is indol-3-yl, which may be substituted in ring position 1 and/or 2 by unsubstituted or phenyl-substituted $C_1$–$C_5$-alkyl; or is thiazol-5-yl which is substituted in ring position 2 by unsubstituted or phenyl-substituted $C_1$–$C_5$-mono- or dialkylamino, pyrrolidino, piperidino or morpholino and may be substituted in ring position 4 by $C_1$–$C_5$-alkyl, phenyl or chlorine, and $R^4$ is hydroxyl, $C_1$–$C_5$-alkoxy, unsubstituted or substituted phenoxy, unsubstituted or substituted phenylsulfonyl, pyrrolidino, piperidino, morpholino or a radical of a C–H-acidic compound, and the ring A may be fused to a benzo ring, may be substituted by $C_1$–$C_4$-alkyl, chlorine or bromine, or may be substituted in ring position 7 by $C_1$–$C_5$-mono- or dialkylamino (each of which in turn may be substituted by chlorine or phenyl), pyrrolidino, piperidino, morpholino, hydroxyl or $C_1$–$C_4$-alkoxy.

2. A process for the preparation of a benzopyran of the formula I as claimed in claim 1, wherein a benzopyrylium of the formula II

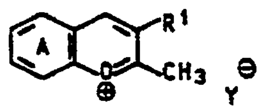

(II)

where $R^1$ and the ring A have the meanings stated in claim 1 and $Y^{\ominus}$ is an anion, is reacted with an aldehyde of the formula III

$R^3$–CHO (III)

where $R^3$ has the meanings stated in claim 1, in the presence of an inert solvent at from 20 to 120°C, and, in a subsequent stage, the resulting dye salt of formula IV

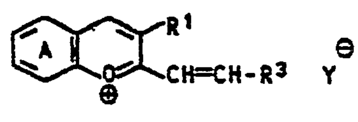

(IV)

where $R^1$, $R^3$, $Y^{\ominus}$ and the ring A have the above meanings, is reacted with a compound of the formula V

$R^4$–H (V)

where $R^4$ has the meanings stated in claim 1, in an inert solvent in the presence of a base at from 20 to 120°C.

3. Use of the benzopyran of the formula I as claimed in claim 1 as a dye precursor in a pressure-sensitive or heat-sensitive recording system.

**Revendications**

1. Benzopyrannes de formule I

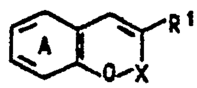

(I),

dans laquelle $R^1$ ist mis pour un radical alkyle en $C_1$–$C_8$ éventuellement substitué, phényle éventuellement substitué, alcoxy en $C_1$–$C_5$ ou halogène et X est mis pour un radical

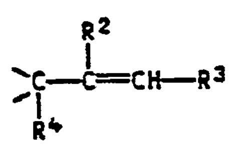 ou 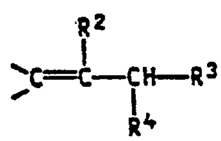

où $R^2$ représente un atome d'hydrogène ou pris avec $R^1$, un groupement alkylène en $C_2$–$C_3$ éventuellement substitué par un radical alkyle en $C_1$–$C_4$,

$R^3$ représente un groupement phényle qui est éventuellement substitué par un radical alkyle en $C_1$–$C_4$ (qui peut lui aussi être substitué par un groupement phényle), cyclohexyle, halogène, alcoxy en $C_1$–$C_8$, mono ou dialkylamino en $C_1$–$C_8$ (qui peut chacun aussi être substitués par un radical chloro ou phényle), phénylamino (qui peut aussi être substitué par un radical alkyle en $C_1$–$C_8$ sur le noyau phényle ou sur l'atome d'azote), pyrrolidino, pipéridino ou morpholino, et qui est de plus éventuellement substitué par un radical alkyle en $C_1$–$C_5$, (alcoxy en $C_1$–$C_5$)carbonyle, di(alkyl en $C_1$–$C_5$)carbonyle, di(alkyl en $C_1$–$C_5$)aminocarbonyle, pyrrolidinocarbonyle, pipéridinocarbonyle, morpholinocarbonyle, alcoxy en $C_1$–$C_8$

ou halogène; naphtyle éventuellement substitué par un radical alcoxy en $C_1$–$C_4$; carbazole-3-yle ou 1,2,3,4,4a,9a-hexahydrocarbazole-3-yle qui peuvent chacun être substitués sur l'atome d'azote par un radical alkyle en $C_1$–$C_5$ ou benzyle; indole-3-yle qui peut être substitué sur le noyau en position 1 et/ou 2 par des radicaux alkyle en $C_1$–$C_5$ éventuellement phényl-substitués; ou thiazole-5-yle qui est substitué sur le noyau en position 2 par un radical mono- ou di(alkyl en $C_1$–$C_5$)amino éventuellement phényl-substitué, pyrrolidino, pipéridino ou morpholino et en position 4 éventuellement par un radical alkyle en $C_1$–$C_5$, phényle ou chloro; et

$R^4$ représente un groupement hydroxy, alcoxy en $C_1$–$C_5$, phénoxy éventuellement substitué, phénylsulfonyle éventuellement substitué, pyrrolidino, pipéridino, morpholino ou le reste d'un composé à CH acide, et dans laquelle le noyau A peut être condensé à un noyau benzo, substitué par des radicaux alkyle en $C_1$–$C_4$, chloro ou bromo ou, en position 7 du noyau, par un radical mono ou di(alkyl en $C_1$–$C_5$)amino (qui peuvent à chaque fois aussi être substitués par un radical chloro ou phényle), pyrrolidino, pipéridino, morpholino, hydroxy ou alcoxy en $C_1$–$C_4$.

2. Procédé de préparation de benzopyrannes de formule I selon la revendication 1, caractérisé en ce qu'on fait réagir, en présence d'un solvant inerte à une température de 20 à 120°C, un dérivé de benzopyrylium de formule II

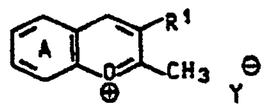

(II),

dans laquelle $R^1$ et le noyau A ont les significations données dans la revendication 1 et $Y^{\ominus}$ représente un anion, avec un aldéhyde de formule III

$R^3$–CHO (III)

dans laquelle $R^3$ a la signification donnée dans la revendication 1, et dans une étape ultérieure on met en réaction, dans un solvant inerte en présence d'une base et à une température de 20 à 120°C, le sel colorant résultant de formule IV

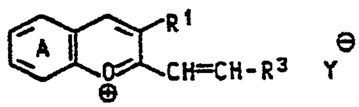

(IV),

dans laquelle $R^1$, $R^3$, $Y^{\ominus}$ et le noyau A ont les significations données précédemment, avec un composé de formule V

$R^4$–H (V)

dans laquelle $R^4$ a la signification donnée dans la revendication 1.

3. Utilisation des benzopyrannes de formule I selon la revendication 1, comme agents chromogènes dans des systèmes d'enregistrement sensibles à la pression ou à la chaleur.